# EUROPÄISCHE PATENTSCHRIFT

(11) **EP 1 119 823 B1**
(45) Veröffentlichungstag und Bekanntmachung des Hinweises auf die Patenterteilung: **05.02.2003**
(21) Anmeldenummer: 99961981.0
(22) Anmeldetag: 12.07.1999
(51) Int. Cl.: G06K 9/00

(54) **BIOMETRISCHER SENSOR UND VERFAHREN ZU DESSEN HERSTELLUNG**
BIOMETRIC SENSOR AND CORRESPONDING PRODUCTION METHOD
DETECTEUR BIOMETRIQUE ET SON PROCEDE DE FABRICATION

(30) Priorität: 14.07.1998 DE 19831570
(43) Veröffentlichungstag der Anmeldung: 01.08.2001
(73) Patentinhaber: Infineon Technologies AG, 81669 München (DE)
(72) Erfinder: FRIES, Manfred, D-94336 Hunderdorf (DE); MÜNCH, Thomas, D-93164 Laaber (DE); FISCHBACH, Reinhard, D-93049 Regensburg (DE)
(74) Vertreter: Hermann, Uwe, Dipl.-Ing.
(86) Internationale Anmeldenummer: DE9902146
(87) Internationale Veröffentlichungsnummer: WO00004491

(56) Entgegenhaltungen:
- EP-A- 0 786 745
- FR-A- 2 736 179
- US-A- 5 483 100

## Beschreibung

Die Erfindung betrifft einen biometrischen Sensor mit einem Sensorchip und einem Chipgehäuse, in das der Sensorchip eingesetzt ist, so wie ein Verfahren zu dessen Herstellung.

Es ist bekannt, personenspezifische Merkmale, beispielsweise Fingerminutien, d.h. Fingerabdrücke, zur Authentifizierung von Personen zu erfassen. Eine derartige Authentifizierung von Personen kann beispielsweise bei Handys, Computer, Kraftfahrzeugen, Schlüsseln, etc. eingesetzt werden. Bei gewissen Anwendungsbereichen, insbesondere bei Handys, ist es erforderlich, das Chipgehäuse möglichst klein zu gestalten, um einen Einbau zu ermöglichen. Insbesondere ist hierbei eine minimale Bauteildicke wünschenswert.

Aus der EP 0 789 334 A2 ist ein biometrischer Sensor bekannt, bei dem ein Sensorchip auf einem Leadframe aufgebracht ist, wobei der Sensorchip mittels eines Wirebonding-Verfahrens elektrisch kontaktiert und mittels einer Mold-Masse derart eingekapselt ist, daß die Sensorfläche durch eine entsprechende Aussparung in der Mold-Masse hindurch zugänglich ist.
Nachteilig ist bei diesem bekannten Sensor, daß er einerseits noch relativ groß gebaut und andererseits relativ kompliziert herzustellen ist.

Der Erfindung liegt daher die Aufgabe zugrunde, einen biometrischen Sensor der eingangs genannten Art zu schaffen, der eine möglichst geringe Größe aufweist und einfach zu fertigen ist. Außerdem soll ein Verfahren zur möglichst einfachen Herstellung eines derartigen biometrischen Sensors geschaffen werden.

Diese Aufgabe wird erfindungsgemäß durch die Merkmale des Anspruchs 1 bzw. 6 oder 9 gelöst. Vorteilhafte Ausführungsformen der Erfindung sind in den weiteren Ansprüchen beschrieben.

Der erfindungsgemäße biometrische Sensor ist durch folgende Merkmale gekennzeichnet:
- der Sensorchip weist Anschlußkontakte in der Form von elektrisch leitfähigen Bumps auf,
- auf der Oberseite des Sensorchips befindet sich eine Kratzschutzabdeckung,
- die Bumps kontaktieren Anschlußleitungen des Chipgehäuses, die im oder am Chipgehäuse vorgesehen sind,
- zwischen der Kratzschutzabdeckung und dem Chipgehäuse befindet sich zumindest um die Sensorfläche herum eine adhäsive Schicht, deren Dicke derart auf die Höhe der Bumps abgestimmt ist, daß eine dichte Verbindung zwischen Sensorchip und Chipgehäuse geschaffen wird.

Für den erfindungsgemäßen biometrischen Sensor ist es charakteristisch, daß das Chipgehäuse bereits an vorbestimmten Stellen Anschlußkontakte für den Sensorchip aufweist. Der Chip wird beim Einsetzen in das Chipgehäuse mit den sich an entsprechenden Stellen befindenden Bumps (höckerartige Erhebungen aus leitfähigem Material) automatisch mit den Anschlußkontakten des Chipgehäuses in Verbindung gebracht, so daß ein nachfolgendes Wirebonden vollständig entfällt. Der Sensorchip sitzt fest und dicht im Chipgehäuse, so daß auch auf das Molden, d.h. Einkapseln des Chips mit einer Kunststoffmasse, vollständig verzichtet werden kann. Weiterhin kann der erfindungsgemäße biometrische Sensor auf sehr einfache, schnelle und ausschußarme Weise hergestellt werden. Ein besonderer Vorteil ist hierbei, daß das Kontaktieren der Bumps mit den Anschlußkontakten des Chipgehäuses und die Abdichtung zwischen dem Chipgehäuse und dem Sensorchip mittels der adhäsiven Schicht in einem Arbeitsschritt erfolgt. Da der erfindungsgemäße Sensorchip auch nicht von beiden Seiten eingekapselt werden muß und das separate Chipgehäuse genau auf die Geometrie des Sensorchips und den späteren Einsatzbereich des Sensors abgestimmt werden kann, kann der erfindungsgemäße Sensor bei vorgegebener Sensorfläche auch wesentlich kleiner als bisher übliche Sensoren hergestellt werden.

Gemäß einer vorteilhaften Ausführungsform ist zusätzlich zu den mit den Anschlußleitungen in Kontakt stehenden Bumps mindestens ein weiterer Auflagebump an der Oberseite des Sensorchips an einer das Kippen des Sensorchips relativ zum Chipgehäuse verhindernden Stelle vorgesehen. Ein derartiger "Dummy-Bump" stellt sicher, daß der Sensorchip nach dem Einsetzen in die entsprechende Vertiefung des Chipgehäuses koplanar zum Chipgehäuse ausgerichtet ist. Hierdurch wird die einwandfreie Kontaktierung sämtlicher Bumps des Sensorchips mit den entsprechenden Anschlußkontakten der Chipgehäuse-Anschlußleitungen sichergestellt.

Zweckmäßigerweise besteht die adhäsive Schicht aus einer rahmenförmigen, umlaufenden Klebefolie, die rings um die Sensorfläche des Sensorchips aufgebracht wird.

Gemäß einer vorteilhaften Ausführungsform besteht das Chipgehäuse aus einem Spritzgußgehäuse, wobei die Anschlußleitungen im Material des Chipgehäuses eingebettet und zu einem äußeren Rand des Chipgehäuses geführt sind. Hierdurch kann eine spätere Beschädigung der Anschlußleitungen vermieden werden. Die am äußeren Rand des Chipgehäuses auftretenden Anschlußleitungen bilden die Leads oder Beinchen, die als Steck-, Löt- oder Klemmkontakte ausgeführt sein können.

Das erfindungsgemäße Verfahren zur Herstellung eines biometrischen Sensors gemäß Anspruch 6 ist durch folgende Schritte gekennzeichnet:
a) Aufbringen von leitfähigen Bumps auf Anschlußkontakte von Sensorchips eines Wafers,
b) Abdecken der Waferfrontseite mit einer Kratzschutzabdeckung,
c) Entfernen der Kratzschutzabdeckung von der Bumpoberseite,
d) Vereinzeln der Sensorchips,
e) Aufbringen einer adhäsiven Schicht um die Sensorfläche des Sensorchips herum, deren Dicke derart auf die Höhe der Bumps abgestimmt ist, daß in einem späteren Verfahrensschritt eine dichte Verbindung zwischen Sensorchip und einem Chipgehäuse geschaffen wird,
f) Einbringen des Sensorchips in ein Chipgehäuse, in dem elektrische Anschlußleitungen vorgesehen sind, wobei die Verklebung des Sensorchips mit dem Chipgehäuse und die Kontaktierung der Bumps mit den Anschlußleitungen des Chipgehäuses gleichzeitig durchgeführt werden.

Für das erfindungsgemäße Verfahren ist es somit charakteristisch, daß bereits im Waferherstellungsprozeß entsprechende Bumps, d.h. höckerartige leitfähige Erhebungen, auf die Sensorchip-Anschlußkontakte des Wafers aufgebracht werden. Dies kann beispielsweise mittels Siebdruck erfolgen. Anschließend wird die Oberseite (Frontseite) des Wafers mit einer vorzugsweise transparenten Kratzschutzabdeckung versehen, deren Schichtdicke auf die Bumphöhe abgestimmt ist. Die Bumps werden dann, vorzugsweise mit einem chemisch-mechanischen Polierverfahren (CMP), von der Kratzschutzabdeckung und einer gegebenenfalls vorhandenen Oxidschicht befreit. Gleichzeitig erreicht man durch diesen Schritt eine Egalisierung der Bumps, d.h. es wird eine Koplanarität zwischen den Bumps hergestellt, wodurch die Kontaktierbarkeit in den nachfolgenden Prozessen verbessert wird. Nach dem Vereinzeln der Sensorchips wird um die Sensorfläche herum ein adhäsives Medium, vorzugsweise eine Klebefolie, aufgebracht, deren Dicke mit der Bumphöhe abgestimmt ist. Der Sensorchip wird nun in ein den Produktanforderungen entsprechendes Gehäuse, vorzugsweise in ein spritzgegossenes Kunststoffgehäuse, montiert, wobei die Verklebung des Sensorchips im Chipgehäuse und die elektrische Kontaktierung zwischen Bumps und Chipgehäuse in einem Schritt erfolgt.

Beim erfindungsgemäßen Verfahren wird somit die Egalisierung der Bumps zum Erreichen einer Koplanarität und das Entfernen der Schutzschicht sowie der Oxidschicht auf Waferebene durchgeführt. Das Kontaktieren der Bumps mit dem Chipgehäuse und die Abdichtung zwischen Chipgehäuse und Sensorchip erfolgt in einem einzigen Arbeitsschritt. Auf diese Weise kann der Sensor auf sehr einfache und kostengünstige Weise und mit sehr geringen Abmessungen hergestellt werden.

Alternativ zu dem Verfahren, bei dem die gesamte Waferfrontseite mit einer Kratzschutzabdeckung abgedeckt und die Kratzschutzabdeckung im Nachhinein von der Bumpoberseite wieder entfernt wird, ist es auch möglich, die Kratzschutzabdeckung derart auf die Waferfrontseite aufzubringen, daß mittels einer geeigneten Maskierung im Bereich der Bumps Öffnungen in der Kratzschutzabdeckung freigehalten werden, wobei die Maske anschließend wieder entfernt wird.

Die Erfindung wird nachfolgend anhand der Zeichnungen beispielhaft näher erläutert. In diesen zeigen:
- Figur 1:: eine Explosionsdarstellung des erfindungsgemäßen biometrischen Sensors,
- Figur 2:: eine teilweise aufgeschnittene perspektivische Ansicht des Sensors von Figur 1,
- Figur 3:: einen vertikalen Längsschnitt des Sensors von Figur 2 längs der Linie A-A von Figur 4,
- Figur 4:: eine Draufsicht auf den Sensor von Figur 2 und
- Figur 5:: eine vergrößerte Darstellung eines vertikalen Längsschnitts des Sensors von Figur 2.

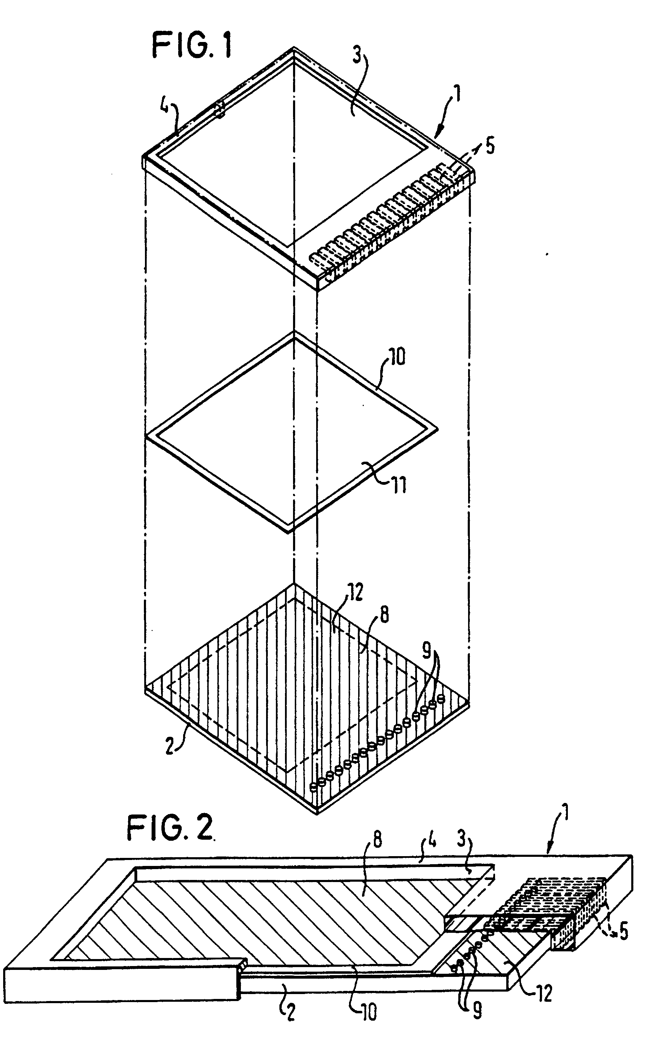

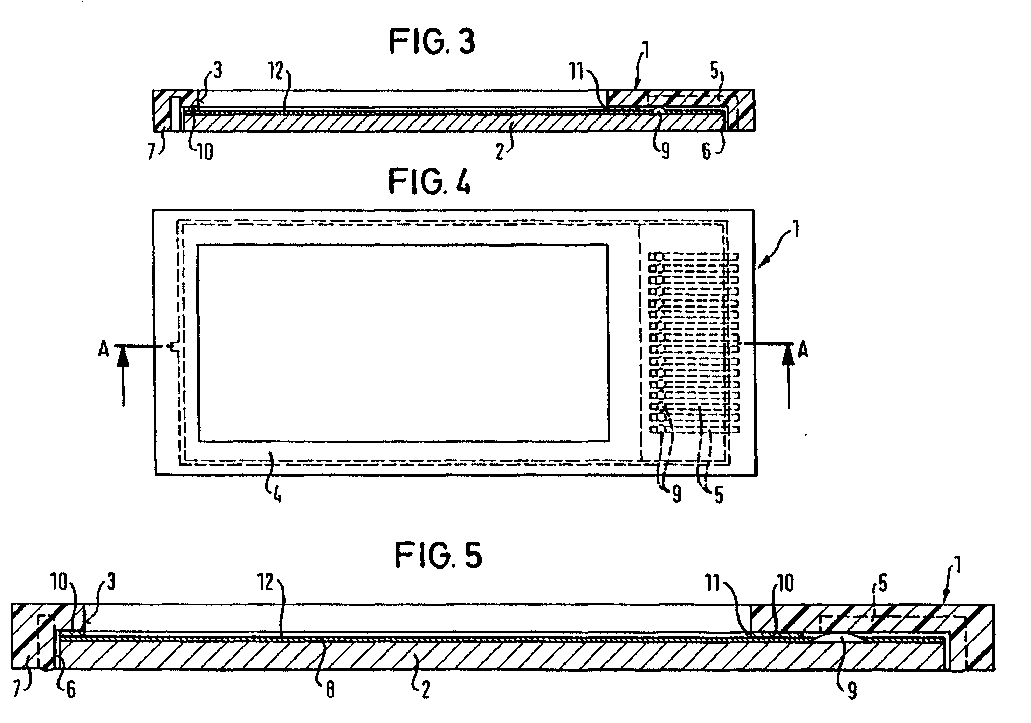

Aus den Figuren 1 bis 5 ist ein biometrischer Sensor ersichtlich, der im wesentlichen aus einem Chipgehäuse 1 und einem Sensorchip 2 besteht.

Das Chipgehäuse 1 besteht aus einem in der Draufsicht rechteckigen Spritzgußgehäuse aus Kunststoff. Eine ebenfalls rechteckige, durchgehende Aussparung 3 des Chipgehäuses 1 weist eine derartige Größe auf, daß der vordere Teil eines Fingers, auf dem sich die Fingerminutien befinden, eingelegt werden kann. Weiterhin ist die Aussparung 3 derart dimensioniert, daß im vorderen Randbereich und in beiden seitlichen Randbereichen nur ein schmaler Steg 4 vorhanden ist. Der hintere Randbereich des Chipgehäuses 1 ist breiter ausgeführt und weist eine Vielzahl nebeneinander liegender Anschlußleitungen 5 auf, die bereits im Chipgehäuse 1 integriert sind. Diese Anschlußleitungen 5 verlaufen in dem gezeigten Ausführungsbeispiel an der Unterseite des Chipgehäuses 1, können jedoch auch vollständig in das Gehäusematerial eingebettet sein, so daß nur ihre vorderen und hinteren Enden zum Zwecke der Kontaktierung freiliegen.

Von der Unterseite her ist im Chipgehäuse 1 eine mittige Vertiefung 6 eingebracht, so daß ein nach unten vorstehender, umlaufender, schmaler Rand 7 gebildet wird, der den eingesetzten Sensorchip 2 seitlich vollständig umschließt. Im eingesetzten Zustand verläuft die Unterseite des Sensorchips 2 fluchtend zur Unterseite des umlaufenden Rands 7. Wie ersichtlich, ist die Breite des Rands 7 auch an der vorderen und hinteren Stirnseite des Chipgehäuses 1 relativ schmal, so daß die Gesamtlänge des Chipgehäuses 1 nur wenig länger als diejenige des Sensorchips 2 ist.

Der Sensorchip 2 weist ein rechteckiges Sensorfeld 8 auf, dessen Größe etwa derjenigen der Aussparung 3 des Chipgehäuses 1 entspricht. Im eingesetzten Zustand des Sensorchips 2 ist das Sensorfeld 8 zur Aussparung 3 des Chipgehäuses 1 ausgerichtet, so daß eine maximale Sensorfeldfläche zum Auswerten der Minutien eines aufgelegten Fingers benutzt werden kann.

Die vom Sensorfeld 8 wegführenden, nicht näher dargestellten Anschlußleitungen enden an der Oberseite des Sensorchips 2 in einer Vielzahl in einer Reihe nebeneinander liegender Anschlußkontakte, die in der Form von elektrisch leitenden Bumps 9, d.h. höckerartigen Erhebungen, ausgebildet sind. Diese Bumps 9 können beispielsweise mittels Siebdruck auf den Wafer aufgebracht werden. Weiterhin sind die Bumps 9 derart angeordnet, daß im eingesetzten Zustand des Sensorchips 2 jeder Bump 9 mit einer zugeordneten Anschlußleitung 5 des Chipgehäuses 1 in Kontakt gelangt, wodurch die elektrische Verbindung zu den Anschlußleitungen 5 hergestellt wird.

Auf der Oberseite des Sensorchips 2 befindet sich weiterhin eine in den Figuren 1 und 2 schraffiert dargestellte, transparente Kratzschutzabdeckung 12, die bereits bei der Waferherstellung vollflächig auf den Sensorchip 2 aufgebracht wird. Die Höhe dieser Kratzschutzabdeckung 12 ist auf die Höhe der Bumps 9 abgestimmt. Weiterhin ist die Kratzschutzabdeckung 12 sowie eine gegebenenfalls vorhandene Oxidschicht beispielsweise mittels eines chemisch-mechanischen Polierverfahrens von der Oberseite der Bumps 9 wieder entfernt worden, so daß eine elektrisch leitfähige Verbindung zwischen den Bumps 9 und den Anschlußleitungen 5 hergestellt werden kann.

Um ein Verkippen des Sensorchips 2 innerhalb des Chipgehäuses 1 bei der Montage zu vermeiden, können im vorderen Endbereich des Sensorchips 2 Auflagebumps vorgesehen sein, die den Bumps 9 entsprechen, jedoch keine elektrische Leitfunktion, sondern nur eine Stützfunktion haben.

Auf dem aus dem Wafer vereinzelten Sensorchip 2 bzw. auf der Kratzschutzabdeckung 12 wird eine adhäsive Schicht 10 in der Form einer rahmenförmigen Klebefolie aufgebracht. Ein mittiger Freiraum 11 der adhäsiven Schicht 10 entspricht der Größe des Sensorfelds 8 bzw. derjenigen der Chipgehäuse-Aussparung 3. Weiterhin ist die Dicke der adhäsiven Schicht 10 auf die Höhe der Bumps 9 abgestimmt. Die adhäsive Schicht 10 dient zum Einkleben des Sensorchips 2 im Chipgehäuse 1. Gleichzeitig wird durch die adhäsive Schicht 10 ein umlaufender Dichtrahmen geschaffen, der eine umlaufende, dichte Verbindung zwischen dem Chipgehäuse 1 und dem Sensorchip 2 gewährleistet.

Wird der Sensorchip 2 zum Zwecke des Verklebens in die Vertiefung 6 des Chipgehäuses 1 eingesetzt, erfolgt gleichzeitig die direkte elektrische Kontaktierung der Bumps 9 mit den Anschlußleitungen 5 des Chipgehäuses 1 sowie die Abdichtung zwischen Sensorchip 2 und Chipgehäuse 1.

## Patentansprüche

1. Biometrischer Sensor mit einem Sensorchip (2) und einem Gehäuse (1), in das der Sensorchip (2) eingesetzt ist, **gekennzeichnet durch** folgende Merkmale:
- der Sensorchip (2) weist Anschlußkontakte in der Form von elektrischen leitfähigen Bumps (9) auf,
- auf der Oberseite des Sensorchips (2) befindet sich eine Kratzschutzabdeckung (12),
- die Bumps (9) kontaktieren Anschlußleitungen (5) des Chipgehäuses (1), die im oder am Chipgehäuse (1) vorgesehen sind,
- zwischen der Kratzschutzabdeckung (12) und dem Chipgehäuse (1) befindet sich zumindest um das Sensorfeld (8) herum eine adhäsive Schicht (10), deren Dicke derart auf die Höhe der Bumps (9) abgestimmt ist, daß eine dichte Verbindung zwischen Sensorchip (2) und Chipgehäuse (1) geschaffen wird.

2. Sensor nach Anspruch 1, **dadurch gekennzeichnet, daß** zusätzlich zu den mit den Anschlußleitungen (5) in Kontakt stehenden Bumps (9) mindestens ein weiterer Auflagebump an der Oberseite des Sensorchips (2) an einer das Kippen des Sensorchips (2) relativ zum Chipgehäuse (1) verhindernden Stelle vorgesehen ist.

3. Sensor nach Anspruch 1 oder 2, **dadurch gekennzeichnet, daß** die adhäsive Schicht (10) aus einer rahmenförmigen Klebefolie besteht.

4. Sensor nach einem der vorhergehenden Ansprüche, **dadurch gekennzeichnet, daß** das Chipgehäuse (1) aus einem Spritzgußgehäuse besteht.

5. Sensor nach einem der vorhergehenden Ansprüche, **dadurch gekennzeichnet, daß** die Anschlußleitungen (5) im Material des Chipgehäuses eingebettet und zu einem äußeren Rand des Chipgehäuses (1) geführt sind.

6. Verfahren zur Herstellung eines biometrischen Sensors, **gekennzeichnet durch** folgende Schritte:
a) Aufbringen von leitfähigen Bumps (9) auf Anschlußkontakte von Sensorchips (2) eines Wafers,
b) Abdecken der Waferfrontseite mit einer Kratzschutzabdeckung (12),
c) Entfernen der Kratzschutzabdeckung (12) von der Bumpoberseite,
d) Vereinzeln der Sensorchips (2),
e) Aufbringen einer adhäsiven Schicht (10) um das Sensorfeld (8) des Sensorchips (2) herum, deren Dicke derart auf die Höhe der Bumps (9) abgestimmt ist, daß in einem späteren Verfahrensschritt eine dichte Verbindung zwischen Sensorchip (2) und einem Chipgehäuse (1) geschaffen wird,
f) Einbringen des Sensorchips (2) in das Chipgehäuse (1), in oder an dem elektrische Anschlußleitungen (5) vorgesehen sind, wobei die Verklebung des Sensorchips (2) mit dem Sensorgehäuse (1) und die Kontaktierung der Bumps (9) mit den Anschlußleitungen (5) des Chipgehäuses (1) gleichzeitig durchgeführt werden.

7. Verfahren nach Anspruch 6, **dadurch gekennzeichnet, daß** das Entfernen der Kratzschutzabdeckung (12) von der Bumpoberseite mit einem chemisch-mechanischen Polierfahren (CMP) durchgeführt wird.

8. Verfahren nach Anspruch 6 oder 7, **dadurch gekennzeichnet, daß** die Freilegung der Bumpoberseiten auf dem Wafer derart erfolgt, daß sämtliche freigelegten Bumpoberseiten auf derselben Ebene liegen.

9. Verfahren zur Herstellung eines biometrischen Sensors, **gekennzeichnet durch** folgende Schritte:
a) Aufbringen von leitfähigen Bumps (9) auf Anschlußkontakte von Sensorchips (2) eines Wafers,
b) Aufbringen einer Kratzschutzabdeckung (12) auf die Waferfrontseite, wobei mittels Maskierung im Bereich der Bumps (9) Öffnungen in der Kratzschutzabdeckung (12) freigehalten werden,
c) Vereinzeln der Sensorchips (2),
d) Aufbringen einer adhäsiven Schicht (10) um das Sensorfeld (8) des Sensorchips (2) herum, deren Dicke derart auf die Höhe der Bumps (9) abgestimmt ist, daß in einem späteren Verfahrensschritt eine dichte Verbindung zwischen Sensorchip (2) und einem Chipgehäuse (1) geschaffen wird,
e) Einbringen des Sensorchips (2) in das Chipgehäuse (1), in oder an dem elektrische Anschlußleitungen (5) vorgesehen sind, wobei die Verklebung des Sensorchips (2) mit dem Sensorgehäuse (1) und die Kontaktierung der Bumps (9) mit den Anschlußleitungen (5) des Chipgehäuses (1) gleichzeitig durchgeführt werden.

## Claims

1. Biometric sensor having a sensor chip (2) and a housing (1), into which the sensor chip (2) is inserted, **characterized by** the following features:
- the sensor chip (2) has connecting contacts in the form of electrically conductive bumps (9),
- on the top of the sensor chip (2) there is a scratch protection covering (12),
- the bumps (9) make contact with connecting leads (5) which belong to the chip housing (1) and are provided in or on the chip housing (1),
- between the scratch protection covering (12) and the chip housing (1) there is, at least around the sensor field (8), an adhesive layer (10), whose thickness is matched to the height of the bumps (9) in such a way that a leaktight connection between sensor chip (2) and chip housing (1) is created.

2. Sensor according to Claim 1, **characterized in that** in addition to the bumps (9) in contact with the connecting leads (5), at least one further supporting bump is provided on the top of the sensor chip (2) at a point which prevents the sensor chip (2) tilting relative to the chip housing (1).

3. Sensor according to Claim 1 or 2, **characterized in that** the adhesive layer (10) consists of a frame-like adhesive film.

4. Sensor according to one of the preceding claims, **characterized in that** the chip housing (1) consists of an injection-moulded housing.

5. Sensor according to one of the preceding claims, **characterized in that** the connecting leads (5) are embedded in the material of the chip housing and are routed to an outer edge of the chip housing (1).

6. Method of producing a biometric sensor, **characterized by** the following steps:
a) applying conductive bumps (9) to connecting contacts belonging to sensor chips (2) of a wafer,
b) covering the front side of the wafer with a scratch protection covering (12),
c) removing the scratch protection covering (12) from the top of the bumps,
d) separating the sensor chips (2),
e) applying an adhesive layer (10) around the sensor field (8) of the sensor chip (2), its thickness being matched to the height of the bumps (9) such that, in a subsequent method step, a leaktight connection between sensor chip (2) and a chip housing (1) is created,
f) introducing the sensor chip (2) into the chip housing (1), in or on which electrical connecting leads (5) are provided, the adhesive bonding of the sensor chip (2) to the sensor housing (1) and the making of contact between the bumps (9) and the connecting leads (5) belonging to the chip housing (1) being carried out simultaneously.

7. Method according to Claim 6, **characterized in that** the scratch protection covering (12) is removed from the top of the bumps by using a chemical mechanical polishing process (CMP).

8. Method according to Claim 6 or 7, **characterized in that** the tops of the bumps on the wafer are exposed in such a way that all the exposed bump tops lie in the same plane.

9. Method of producing a biometric sensor, **characterized by** the following steps:
a) applying conductive bumps (9) to connecting contacts belonging to sensor chips (2) of a wafer,
b) covering the front side of the wafer with a scratch protection covering (12), openings in the scratch protection covering (12) being kept free by means of masking in the area of the bumps (9),
c) separating the sensor chips (2),
d) applying an adhesive layer (10) around the sensor field (8) of the sensor chip (2), its thickness being matched to the height of the bumps (9) such that, in a subsequent method step, a leaktight connection between sensor chip (2) and a chip housing (1) is created,
e) introducing the sensor chip (2) into the chip housing (1), in or on which electrical connecting leads (5) are provided, the adhesive bonding of the sensor chip (2) to the sensor housing (1) and the making of contact between the bumps (9) and the connecting leads (5) belonging to the chip housing (1) being carried out simultaneously.

## Revendications

1. Détecteur biométrique comprenant une puce (2) et un boîtier (1) dans lequel la puce (2) est insérée, **caractérisé par** les caractéristiques suivantes :
- la puce (2) présente des contacts de raccordement sous la forme de bossages (9) conducteurs électriques,
- sur la face supérieure de la puce (2) se trouve un film de protection vis-à- vis des rayures(12),
- les bossages (9) sont en contact avec les branchements (5) du boîtier (1), de puce qui sont prévus dans ou sur le boîtier (1) de puce,
- entre le film de protection (12) vis-à-vis des rayures et le boîtier (1) de puce se trouve au moins une couche adhésive (10) autour de la zone (8) de détecteur, dont l'épaisseur est adaptée à la hauteur des bossages (9), de sorte qu'une liaison étanche entre la puce (2) de détecteur et le boîtier (1) de puce est créée.

2. Détecteur selon la revendication 1, **caractérisé en ce que**, outre les bossages (9) existants en contact avec les branchements (5), au moins un autre bossage d'appui est prévu sur la surface supérieure de la puce (2) de détecteur à un emplacement empêchent le basculement de la puce (2) de détecteur par rapport au boîtier (1), de puce.

3. Détecteur selon la revendication 1 ou 2, **caractérisé en ce que** la couche adhésive (10) est composée d'un ruban adhésif ayant la forme d'un cadre.

4. Détecteur selon l'une quelconque des revendications précédentes, **caractérisé en ce que** le boîtier (1) de puce est un boîtier moulé par injection.

5. Détecteur selon l'une quelconque des revendications précédentes, **caractérisé en ce que** les branchements (5) sont insérés dans le matériau du boîtier de puce et vont jusqu'à un bord extérieur du boîtier (1) de puce.

6. Procédé de fabrication d'un détecteur biométrique **caractérisé par** les étapes suivantes :
a) Dépôt de bossages (9) conducteurs sur les contacts de raccordement de la puce (2) de détecteur d'une plaquette,
b) Recouvrement de la face avant de la plaquette avec un film de protection vis-à-vis des rayures (12),
c) Suppression du film de protection via-à-vis des rayures (12) de la face supérieure des bossages,
d) Individualisation des puces (2) de détecteur
e) Dépôt d'une couche adhésive (10) entourant la zone de détecteur (8) de la puce (2) de détecteur, dont l'épaisseur est adaptée à la hauteur des bossages (9), de sorte que dans une étape suivante du procédé, une connexion étanche entre la puce (2) de détecteur et un boîtier (1) de puce est créée,
f) Introduction de la puce (2) de détecteur dans le boîtier (1), de puce dans lequel ou sur lequel des branchements électriques (5) sont prévus, le collage de la puce (2) de détecteur au boîtier (1) de puce et la mise en contact des bossages (9) avec les branchements (5) du boîtier (1) de détecteur étant effectués en même temps.

7. Procédé selon la revendication 6, **caractérisé en ce que** la suppression du film de protection vis-à-vis des rayures (12) de la surface supérieure des bossages est effectuée par un procédé de polissage chimique et mécanique (CMP).

8. Procédé selon la revendication 6 ou 7, **caractérisé en ce que** l'on effectue la mise à nu des faces supérieures des bossages sur la plaquette, de sorte que toutes les surfaces supérieures des bossages se trouvent au même niveau.

9. Procédé de fabrication d'un détecteur biométrique **caractérisé par** les étapes suivantes :
a) dépôt de bossages (9) conducteurs sur les contacts de raccordement de la puce (2) de détecteur d'une plaquette.
b) dépôt d'un film de protection vis-à-vis des rayures (12) sur la face avant de la plaquette, des ouvertures étant dégagées dans le film de protection vis-à-vis des rayures (12) au moyen d'un masque dans la zone des bossages (9),
c) individualisation des puces (2) de détecteur
d) dépôt d'une couche adhésive (10) entourant la zone de détecteur 8) de la puce (2) de détecteur, dont l'épaisseur est adaptée à la hauteur des bossages (9), de sorte que dans une étape suivante du procédé, une connexion étanche entre la puce (2) de détecteur et un boîtier (1) de puce est créée,
e) mise en place de la puce (2) de détecteur dans le boîtier (1) de puce dans lequel ou sur lequel des branchements électriques (5) sont prévus, le collage de la puce (2) de détecteur avec le boîtier (1) de puce et la mise en contact des bossages (9) avec les branchements (5) du boîtier (1) de puce étant effectués en même temps.
